(19)

**Europäisches Patentamt**

**European Patent Office**

**Office européen des brevets**

(11) **EP 3 505 891 A1**

(12) **EUROPEAN PATENT APPLICATION**

(43) Date of publication:
**03.07.2019 Bulletin 2019/27**

(51) Int Cl.:
**G01K 7/42** (2006.01)　　　**A61B 5/01** (2006.01)
**G01K 13/00** (2006.01)

(21) Application number: **17390001.0**

(22) Date of filing: **29.12.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD TN**

(71) Applicant: **Medectis IP Ltd.**
**3022 Limassol (CY)**

(72) Inventors:
• **Kounoudes, Anastasis**
**4106 Limassol (CY)**

• **Constantinides, Anthony George**
**London N10 3AN (GB)**

(74) Representative: **Röthinger, Rainer**
**Wuesthoff & Wuesthoff**
**Patentanwälte PartG mbB**
**Schweigerstrasse 2**
**81541 München (DE)**

Remarks:
•Amended claims in accordance with Rule 137(2) EPC.
•The references to the drawing(s) no. 8-12 are deemed to be deleted (Rule 56(4) EPC).

(54) **NON-INVASIVE TECHNIQUE FOR BODY CORE TEMPERATURE DETERMINATION**

(57)　　An apparatus is presented for determining a body core temperature from temperature measurements taken by a device that is configured to be brought into skin contact and that is associated with at least one device parameter indicative of a thermal device characteristic. The apparatus is configured to determine a series of temperature measurements taken between a first point in time when or after the device is brought into skin contact and a second point In time before or when a substantially constant temperature is measured, wherein the temperature measurements have been taken at associated measurement times. The apparatus is further configured to determine the body core temperature at least from the series of temperature measurements, the associated measurement times and the at least one device parameter.

FIG. 4

**Description**

**Technical Field**

The present disclosure generally relates to a non-invasive technique for determining a body core temperature from temperature measurements at a skin surface. The technique may be implemented in the form of an apparatus, a method or a computer program product.

**Background**

Body core temperature measurements can be taken using a variety of thermometer types. Conventional mercury-based thermometers have been used for a long time. While allowing precise measurements, such thermometers are inconvenient to use for both the patient and the medical staff due to their invasiveness.

In-ear thermometers with integrated infrared sensors are increasingly replacing conventional thermometers despite being less accurate. In view of their lacking accuracy, in-ear thermometers are generally not usable for body core temperature determination in clinical environments.

Another thermometer type predicts the body core temperature from skin temperature measurements. This thermometer type can easily be attached to a patient's skin and, thus, is more convenient for the patient and also easier to use from the perspective of the medical staff. Moreover, the precision of body core temperature determination from skin temperature measurements can reach a sufficiently high precision for clinical environments.

Solman et al., "New thermometers for deep tissue temperature", Biomedical Engineering, October 1973, 432-435 and Fox et a., "A new technique for monitoring deep body temperature from the skin surface", Clin. Sci. 1973, 44, 81-86 describe a non-invasive technique for body core temperature determination from skin temperature measurements using the two thermistors, a layer of thermally insulating material separating the two thermistors and a heater. A first one of the two thermistors is intended to be brought into skin contact while the second thermistor on the opposite side of the thermally insulating material is exposed, via a further separating layer of thermally insulating material, to the heater. The heater is controlled such that an outward flow of heat from deep body tissue is matched by an inward flow of heat coming from the heater (as measured by the first and second thermistors, respectively). Once an equilibrium state has been reached, the body core temperature can accurately be determined from the measured parameters. In practice It takes, however, some 20 minutes to reach the equilibrium state. Moreover, the heater consumes a considerable amount of electrical power so that the device cannot perform long-term measurements without being connected to an external power source.

More recently, another non-invasive technique for body core temperature determination from skin temperature measurements has been described in Kitamura et al., "Development of a new method for the noninvasive measurement of deep body temperature without a heater", Medical Engineering & Physics 32 (2010) 1-6. This technique uses four thermistors but does not require a heater. As such, the power consumption can considerably be decreased. On the other hand, the period of time to reach the thermal equilibrium is significantly Increased to up to 30-40 minutes, which means that a reliable body core temperature value is only obtained 30-40 minutes after the point in time at which the device has been attached to the patient.

**Summary**

There is a need for a non-invasive technique that allows a fast and reliable determination of the body core temperature from temperature measurements at the skin surface. The technique should optionally be implementable with few hardware resources and in a power-efficient manner.

According to a first aspect, an apparatus is presented for determining a body core temperature from temperature measurements taken by a device that is configured to be brought Into skin contact and that is associated with at least one device parameter indicative of a thermal device characteristic. The apparatus is configured to determine a series of temperature measurements taken between a first point in time when or after the device is brought into skin contact and a second point in time before or when a substantially constant temperature is measured, wherein the temperature measurements have been taken at associated measurement times. The apparatus is further configured to determine the body core temperature at least from the series of temperature measurements, the associated measurement times and the at least one device parameter.

The device that takes the temperature measurements can comprise the apparatus for determining the body core temperature or may be configured to be located remotely from this apparatus.

A first temperature measured at the first point in time may be lower than a skin temperature. The first temperature may be ambient temperature or may lie in a temperature range above ambient temperature and below skin temperature.

The substantially constant temperature may correspond to skin temperature. The substantial constant temperature may exhibit temperature variations smaller than the temperature difference between the first temperature and skin temperature. The substantial constant temperature may exhibit temperature variations not exceeding skin temperature variations that are due to body core temperature changes.

The apparatus may be configured to determine the body core temperature by applying a temperature change model, such as an asymptotic temperature growth model, to the series of temperature measurements and the associated measurement times. The temperature change model may describe a temporal behavior of the measured temperature between the first point in time and the second point in time. Applying the temperature change model to the series of temperature measurements and the associated measurement times may be performed using a fitting procedure. In this manner, one or more model parameters of the temperature change model may be derived, so that the body core temperature may be determined from the one or more model parameters and the one or more device parameters.

The one or more model parameters of the temperature change model may comprise a model parameter indicative of a time behavior of the temperature change (e.g., between the first point in time and the second point In time). This model parameter may be determined by applying (e.g., fitting) the temperature change model to the series of skin temperature measurements and the associated measurement times.

It will be appreciated that the temperature change model may comprise one or more further model parameters in addition to the model parameter indicative of a time behavior of the temperature change, such as model parameters representing at least one of an initial temperature and a final temperature of the temperature change. At least one of the initial temperature parameter and the final temperature parameter may be determined from the temperature model by fitting. The body core temperature may then also be determined from at least one of the initial temperature parameter and the final temperature parameter.

The initial temperature parameter may be indicative of a temperature that would be measurable at t = 0 before the device is brought into skin contact. The final temperature parameter may be indicative of a temperature that would be measurable at t = ∞. It should be noted that the initial temperature parameter and the final temperature parameter may constitute mere model parameters but no measureable temperatures.

The temperature change model in a time domain representation can be based on the following expression:

$$T(t) = Tinf + (T0 - Tinf) * exp(-alpha * t),$$

wherein $T(t)$ is the temperature measurement at measurement time $t$, alpha is the model parameter indicative of the time behavior of the temperature change, T0 is the initial temperature parameter and Tinf Is the final temperature parameter. It should be noted that the temperature change model need not necessarily be applied in its time domain representation. As an example, also the Laplace transform of the temperature change model may be applied.

In one variant, the body core temperature Tc is determined, or modeled, as a function of the device parameter, the model parameter indicative of a time behavior of the temperature change, the initial temperature parameter and the final temperature parameter. As an example, the body core temperature Tc may be determined based on the following expression:

$$Tc = f(Ca; alpha; T0; Tinf),$$

wherein Ca is the device parameter, alpha is the model parameter indicative of the time behavior of the temperature change, T0 is the initial temperature parameter and Tinf is the final temperature parameter.

The expression $Tc = f(Ca; alpha; T0; Tinf)$ can thus be considered to represent a body core temperature model. In a more general representation, the body core temperature model can also be expressed as $Tc = f(Ca; alpha)$. Of course, the body core temperature model does not need to include these parameters Ca and alpha (as well as T0 and Tinf in a more concrete variant) as such. Rather, the body core temperature model can also be expressed in terms of other parameters that include, or are derived from, the parameters Ca and alpha (as well as T0 and Tinf in a more concrete variant).

The apparatus according to the first aspect may further be configured to process continued temperature measurements taken after the second point in time to detect a skin temperature change. In such a case the body core temperature may be determined (e.g., updated or recalculated) on the basis of the continued temperature measurements comprising the detected skin temperature change. For example, the body core temperature may initially be determined based on measurements taken in any interval between the first point in time and the second point in time and may then be updated or recalculated after the second point in time based on the continued temperature measurements.

In one implementation, the apparatus is configured to differentiate "actual" skin temperature changes from skin temperature measurement artefacts. To this end, a threshold may be set based on calibrated or predefined characteristics of a temperature sensor (e.g., a thermistor) used to take the temperature measurements. Specifically, the threshold may be set to be sufficiently away from any "built-in" measurement artefacts yielded by the temperature sensor upon measuring a constant temperature (e.g., a constant calibration temperature).

The apparatus may be configured to determine if the detected skin temperature change is due to an external effect different from a body core temperature change. As such, the apparatus may be configured to distinguish if the detected skin temperature change is due to a body core temperature change or to an external effect. The external effect may, for example, result from the patient taking off clothes, which may result in a skin temperature change not stemming from a body core temperature change. The apparatus may be configured to evaluate a temporal behavior of the skin temperature change to determine if the detected skin temperature change is due to an external effect.

The apparatus may be configured to determine that the detected skin temperature change is due to an external effect if a magnitude of the skin temperature change within a given period of time exceeds a predefined temperature change threshold. The apparatus may be configured to newly calculate at least one parameter of the temperature change model (e.g., the model parameter indicative of a time behavior or a model parameter derived therefrom) if the detected skin temperature change is due to an external effect. To this end, the temperature change model may be applied to at least some of the skin temperature measurements from which the external effect has been determined. Moreover, the apparatus may be configured to re-use, for determining the body core temperature from the continued skin temperature measurements, the at least one previously determined parameter of the temperature change model (e.g., the model parameter indicative of a time behavior or a model parameter derived therefrom) if the detected skin temperature change is not due to an external effect.

The apparatus may also be configured to detect that the device has been brought into skin contact. Skin contact detection may be performed by evaluating a temporal behavior of the temperature measurements (starting, e.g., before the first point in time and ending at or after the first point in time). As an example, a detected temperature increase that follows a predefined pattern may signal skin contact. In response to skin contact detection, the device can identify the first point in time (i.e., may determine the beginning of the temperature measurements series underlying body core temperature determination).

Body core temperature determination is based one or more device parameters. The one or more device parameters may be obtained in a calibration procedure preceding the actual temperature measurements for body core temperature determination. The calibration procedure may in particular be performed at a calibration site before delivery of the device to the customers. In one variant, the calibration procedure comprises measuring, by the device, a calibration temperature, wherein the calibration temperature or a temperature difference between the calibration temperature and ambient temperature is known *a priori*. The device parameter may be indicative of a heat capacitance of a device.

According to a second aspect, a method is presented of determining a body core temperature from temperature measurements taken by a device configured to be bought into skin contact and associated with at least one device parameter indicative of a thermal device characteristic. The method comprises determining a series of measurements taken between a first point in time when or after the device is brought into skin contact and a second point in time before or when a substantially constant temperature is measured, wherein the temperature measurements have been taken at associated measurement times. The method further comprises determining the body core temperature at least from the series of the temperature measurements, the associated measurement times and the at least one device parameter.

The method may further comprise one or more additional steps, functions and processes as generally described above and below. The method may in particular comprise operating the apparatus described herein.

According to a third aspect, an apparatus for processing skin temperature measurements to determine a body core temperature from a body core temperature model is provided. The apparatus is configured to process a series of skin temperature measurements taken at associated measurement times to detect a skin temperature change, to determine that the detected skin temperature change is due to an external effect different from a body core temperature change, and to determine at least one model parameter of the body core temperature model so as to compensate for the external effect when determining the body core temperature from further skin temperatures measurements.

The apparatus of the third aspect may further be configured to determine the at least one model parameter by applying a temperature change model to the series of skin temperature measurements from which the external effect was determined. Moreover, a point in time indicative of occurrence of the external event in the series of skin temperature measurements may be determined. The point in time of occurrence of the external event may, for example, be determined from a peak in a running standard deviation (or similar statistic measure) of the continued skin temperature measurements. Moreover, the temperature change model may be applied to a sub-series of the skin temperature measurements preceding that point in time to determine the model parameter that compensates for the external effect when determining the body core temperature from upcoming skin temperature measurements and from the body core temperature model.

The body core temperature model may be expressed as $Tc = f(Ca; alpha)$, wherein $Tc$ is the body core temperature, $Ca$ is a device parameter of a device that has taken the temperature measurements, and alpha is a temperature change model parameter indicative of an ambient heat flow resistance. The ambient heat flow resistance may be a parameter of an environment of the device that has taken the temperature measurements.

A fourth aspect relates to a method for processing skin temperature measurements to determine a body core temperature from a body core temperature model. The method comprises processing a series of skin temperature measurements taken at associated measurement times to detect a skin temperature change, determining that the detected skin temperature change is due to an external effect different from a body core temperature change, and determining at least one model parameter of the body core temperature model so as to compensate for the external effect when determining the body core temperature from further skin temperatures measurements.

According to another aspect, a computer program product is presented comprising program code portions to perform any of the methods and method steps presented herein when the computer program product Is executed by at least one processor. The computer program product may be stored on a computer-readable recording medium, such as a semiconductor memory, CD, DVD, and so on. Moreover, the computer program product may be provided for download via a network.

**Brief Description of the Drawings**

Further aspects, details and advantages of the present disclosure will become apparent from the following description of exemplary embodiments and from the drawings, wherein

Fig. 1     shows a schematic block diagram of a first device embodiment for body core temperature detection;

Fig. 2     shows a schematic block diagram of a second device embodiment for body core temperature determination;

Fig. 3     shows a flow diagram underlying a first method embodiment for body core temperature detection in a transient phase;

Fig. 4     shows a diagram illustrating skin temperature measurement intervals for initial body core temperature detection in a transient phase;

Fig. 5     shows a diagram illustrating skin temperature measurement artefacts after the transient phase;

Fig. 6     shows a diagram illustrating a skin temperature increase due to a body core temperature increase after the transient phase;

Fig. 7     shows a diagram illustrating a skin temperature increase due to an external effect after the transient phase;

Fig. 8     shows a flow diagram underlying a second method embodiment for body core temperature determination after the transient phase;

Fig. 9     shows a diagram illustrating occurrence of a peak in a standard deviation domain due to an external event;

Fig. 10    shows an equivalent circuit underlying a temperature change model according to an embodiment of the present disclosure;

Fig. 11    shows a diagram of skin temperature measurements; and

Fig. 12    shows the process of fitting a temperature change model to the skin temperature measurements of Fig. 11.

**Detailed Description**

The following embodiments relate to concepts, procedures and algorithms for the non-invasive determination of the core body temperature from temperature measurements in skin contact. In some variants, no further parameters need to be measured "on line" for core body temperature determination besides the skin temperature, although "off line" measurements can be performed for calibration purposes.

The framework presented herein in one variant permits to distinguish between two main "events" which may be observed in the measured skin temperature data, namely a core body temperature change on the one hand and an external effect, typically due to an ambient heat flow resistance change, on the other hand. In this regard, use is made of the fact that body core temperature changes are not impulsive and change at a rate of a few degrees Celsius (°C) per hour, whereas ambient heat flow resistance changes are considerably shorter or impulsive in nature.

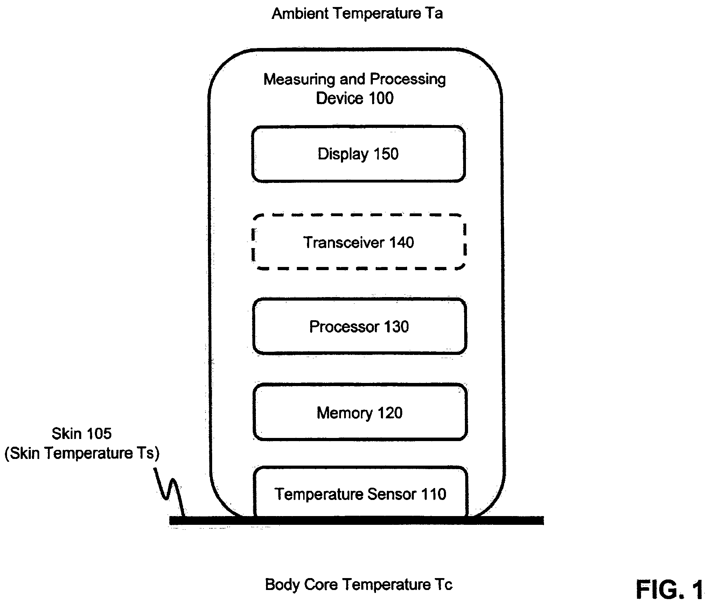

Fig. 1 illustrates a first embodiment of a device 100 for non-invasively determining the core body temperature Tc from measurements in skin contact. The device 100 is configured to be brought into contact with a skin surface 105 via an adhesive patch or otherwise. A certain period of time after the device 100 has been brought into skin contact (i.e., after a transient phase), the actual skin temperature Ts will be measured. On its portion(s) not in skin contact, the device 100 is typically exposed to ambient temperature Ta. The ambient temperature Ta may correspond to room temperature or may be higher (e.g., when the device Is covered by a layer of clothing).

As shown in Fig. 1, the device 100 comprises at least one temperature sensor 110 (e.g., a thermistor) configured to perform temperature measurements. In practice, one temperature sensor 110 will be sufficient for the core body temperature determination approaches described herein. In some use cases (e.g., for clinical use) the device 100 may be equipped with two or more temperature sensors 110 to increase the measurement accuracy. Measurement accuracy can, for example, be increased by averaging or mutually validating the skin temperature measurements of the two or more temperature sensors 110.

The device 100 further comprises at least one memory 120 and at least one processor 130 having access to the at least one memory 120. The at least one memory 120 may be realized as a single memory chip, as two or more memory chips, as a hard disk, or otherwise.

The at least one memory 120 is configured to store temperature measurements taken by the at least one temperature sensor 110 together with the associated measurement times. The temperature measurements and associated measurement times are jointly referred to as temperature measurement data herein. The at least one memory 120 is further configured to store one or more calibration parameters determined "off line" for the device 100 at a calibration site (e.g., at the end of the device manufacturing line). As an example, a calibrated device parameter indicative of a thermal device characteristic, such as an ambient heat capacity or an associated time constant of the device 100, may be stored. Additionally, one or more further device parameters not associated with its thermal properties may be stored (e.g., parameters indicative of a measurement accuracy of the at least one temperature sensor 110).

The at least one memory 120 optionally stores program code that controls the operation of the at least one processor 130. Such program code may, for example, control the at least one processor 130 in the context of receiving temperature measurements from the at least one temperature sensor 110 and writing these measurements and associated measurement times in the at least one memory 120. Moreover, such program code may control the at least one processor 130 in the context of accessing the measurement data in the at least one memory 120 and processing the accessed measurement data for body core temperature determination. In this regard, the at least one processor 130 may store temporary data calculated in connection with body core temperature determination in the at least one memory 120.

The device 100 illustrated in Fig. 1 further comprises an optional transceiver 140. The transceiver may be configured in accordance with a wireless (e.g., Bluetooth or WLAN) or a wirebound (e.g., USB) communication standard.

The transceiver 140 is in one variant configured to transmit the temperature measurement data (e.g., "on line" as taken by the temperature sensor 110 or "off line" as retrieved from the memory 120) to an external device (not shown in Fig. 1). In a second variant that may be combined with the first variant, the transceiver 140 is configured to transmit the body core temperature as determined by the processor 130 to an external device.

The device 100 also comprises a display 150 for displaying the body core temperature as determined by the processor 130. The display 150 may be realized as a liquid crystal display, using (e.g., organic) light emitting diodes, or otherwise. In some use cases the display 150 may be omitted.

Moreover, while not shown in Fig. 1, the device 100 further comprises a battery for powering the components 110 to 150. The battery may be rechargeable. Alternatively, or in addition, the device 100 may be connectable to an external power supply (e.g., via a cable).

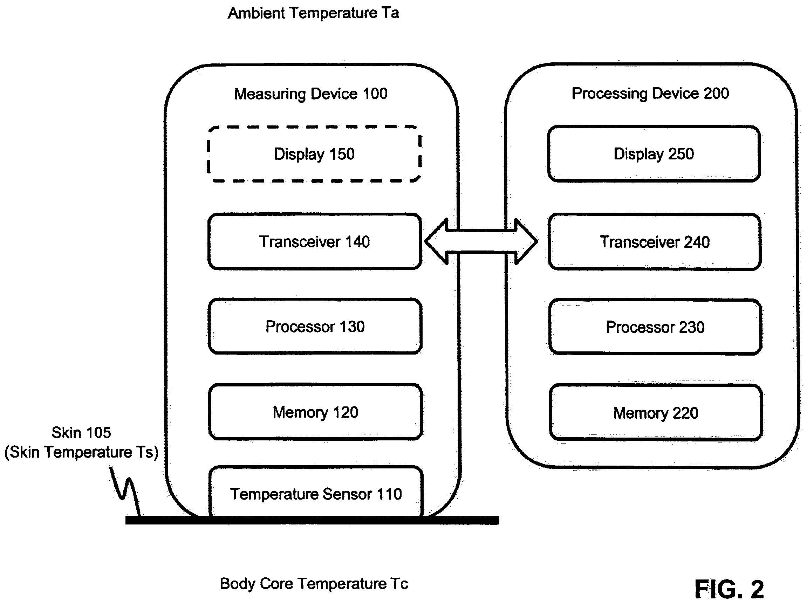

Fig. 2 illustrates a second embodiment of a body core temperature determination device 200. The device 200 is intended to be used in a system together with a measuring device 100. The same reference numerals as in the first device embodiment of Fig. 1 are used to denote the same or similar components.

As shown in Fig. 1, the device 200, similar to the device 100, comprises at least one memory 220, at least one processor 230, a transceiver 240 and a display 250. The device 200 can be a mobile terminal, such as a smartphone, a tablet computer or a laptop. In an alternative configuration (e.g., in a clinical environment), the device 200 can be a stationary component. Moreover, the device 100 could also be configured as a wearable device, such as a smart watch.

The transceiver 240 is in one variant configured to receive from the transceiver 150 of the measuring device 100 the temperature measurement data that have been taken as described above with reference to Fig. 1. In this variant, the measuring device 100 does not need to be equipped with capabilities for core body temperature determination. This means that the processing requirements for the at least one processor 130 can be relaxed compared to the scenario illustrated In Fig. 1, which also reduces the complexity, the power consumption and the costs of the measuring device 100 (as the display 150 can be omitted also).

In the variant in which the device 200 receives via the transceiver 240 the temperature measurement data from the device 100, the received data will be stored in the at least one memory 220. Moreover, also one or more calibration parameters stored for the device 100 in its memory 120 may be received via the transceiver 240 and stored in the at least one memory 220. The one or more calibration parameters may also be received by the device 200 separately from the temperature measurement data (e.g., in an initial pairing or initialization procedure performed between the two devices 100, 200) and either from the device 100 itself or from an external data source different from the device 100.

The at least one memory 220 also stores program code that controls the operation of the at least one processor 230. Such program code specifically controls the at least one processor 230 in connection with accessing the measurement data in the at least one memory 220 and processing the accessed measurement data for body core temperature determination. The at least one processor 230 may store temporary data calculated in connection with body core temperature determination in the at least one memory 220. Once the body core temperature has been determined, It will be displayed at the display 250.

In another variant, the transceiver 240 readily receives the body core temperature from the device 100. The received body core temperature may be stored in the at least one memory 220 and/or displayed at the display 250.

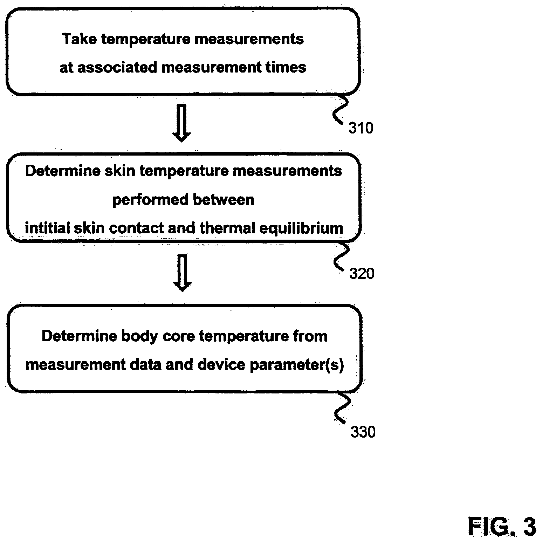

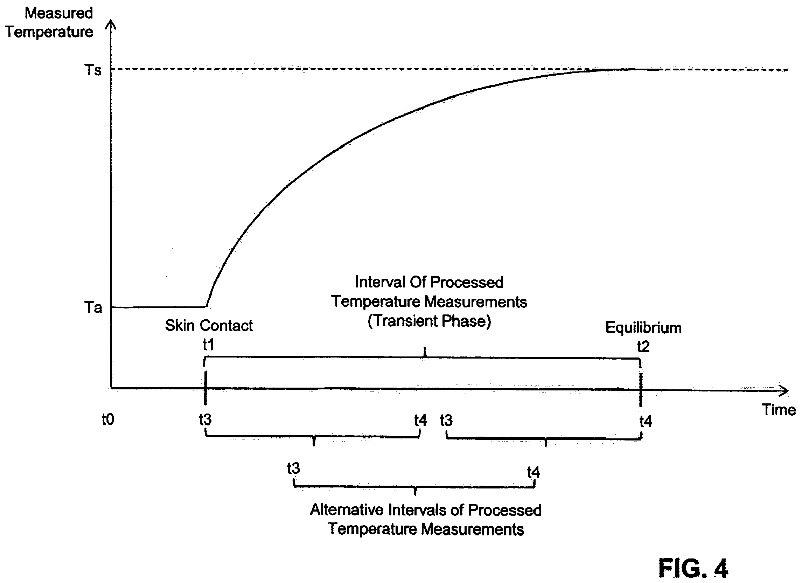

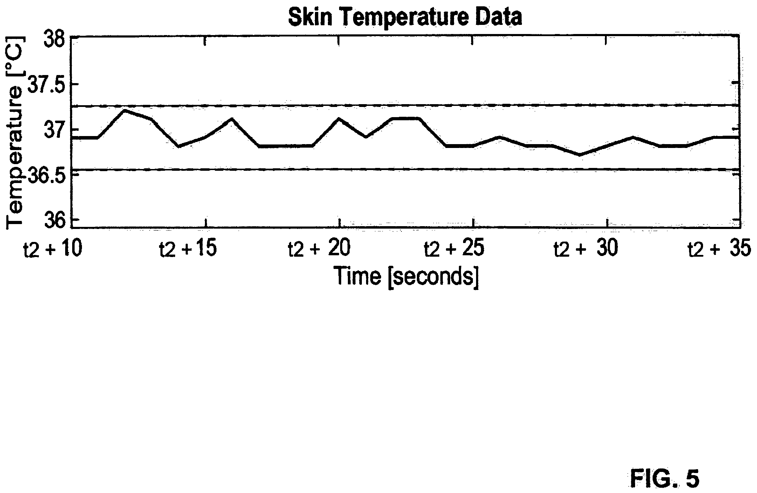

In the following, operation of the device 100 of Figs. 1 and 2 and of the device 200 of Fig. 2 will be described with reference to the flow diagram of Figs. 3 and 5 as well as with reference to the schematic temperature evolution diagram of Fig. 4.

The flow diagram of Fig. 3 Illustrates a method embodiment of non-invasively determining a body core temperature from temperature measurements.

In an initial step 310, temperature measurements are taken by the at least one temperature sensor 110 under control of the processor 130 and stored in the memory 120 together with the associated measurement times. The temperature measurements can be taken continuously (e.g., at a sampling frequency of 1 sample/second or higher). The temperature measurements may automatically start when the device 100 is switched on. At that time, the device 100 may not yet be in skin contact. In other words, the device 100 may be configured to take temperature measurements during a continuous period of time starting before skin contact and not ending after the device 100 has been brought into skin contact.

The temperature measurements acquired in step 310 comprise a series of skin temperature measurements taken in a transient phase between a first point in time when or after the device 100 is brought into skin contact and a second point in time before or when the temperature sensor 110 measures an essentially constant temperature. At the second point in time, the device 100 and the skin surface 105 have substantially the same temperature at their contact surface. This means that starting from the second point in time, the actual skin temperature $T_s$ can be measured. In the transient phase starting at the first point in time and preceding the second point in time, the measured temperature gradually approaches the skin temperature $T_s$ until substantially a thermal equilibrium at the contact area between the skin surface 105 and the device 100 has been reached.

Fig. 4 schematically shows the measured temperature as a function of time and starting with switching on of the device 100 at point in time $t0$. Since at point in time $t0$ the device 100 is not yet in skin contact, it measures the ambient temperature $T_a$.

At point In time $t1$, the device 100 is brought into skin contact. As can be seen, the measured temperature continuously rises from the ambient temperature $T_a$ until reaching substantially an equilibrium state at point in time $t2$, which corresponds to measurement of the actual skin temperature $T_s$.

The series of temperature measurements from which the body core temperature will be determined is defined by an interval start time $t3$ (starting at or after point in time $t1$ when the device 100 is brought in to skin contact) and an interval end time $t4$ (ending before or at the point in time $t2$ when the temperature sensor 110 measures an essentially constant temperature). In Fig. 4, three alternative intervals are shown to demonstrate scenarios in which $t3 > t1$ and $t4 < t2$.

The processor 130 is configured to evaluate the temperature measurements starting at point in time $t0$ to detect skin contact at point in time $t1$. Skin contact detection by the processor 130 may be based on recognizing the characteristic pattern of the temperature increase from ambient temperature $T_a$ at and after point in time $t1$ (i.e., when the device 100 initially comes into thermal contact with the skin surface 105). In the system setup Illustrated in Fig. 2, skin contact detection can also be performed by the processor 230 of the device 200.

The processor 130 is further configured to evaluate the temperature measurements after point in time $t1$ to detect a substantial thermal equilibrium at point in time $t2$. Thermal equilibrium detection by the processor 130 may be based on recognizing the characteristic temperature plateau after point in time $t2$. Detection of the temperature plateau may, for example, be performed by calculating a (e.g., running) variance or standard deviation over the temperature measurements taken after point in time $t1$. When it is determined that the variance or standard deviation falls below a predefined threshold (that may be stored as a calibrated device parameter in the memory 120), the thermal equilibrium is reached. Of course, other statistic measures of the temperature measurements could be evaluated as well in this regard. In the system setup Illustrated In Fig. 2, thermal equilibrium detection can be performed by the processor 230 of the device 200.

Reaching a thermal equilibrium after point in time $t2$ does not mean that the measured skin temperature may not change further (e.g., due to a change of the body core temperature or due to an external effect such as putting on a layer of clothing). However, any skin temperature change after point in time $t2$ will typically have a temporal gradient smaller than the temporal gradient occurring in the transient phase between point in time t1 and point in time t2.

As said, the device 100 continuously takes temperature measurements starting at point in time t0 when the device 100 is switched on, and these measurements will continuously be stored in the memory 120 (or In the memory 220) together with the associated measurement times. Once the points in time t1 and t2 (corresponding to skin contact and thermal equilibrium) have been detected, the processor 130 in step 320 can determine the series of temperature measurements falling in the interval defined by the points in time t1 and t2 (or in a sub-interval defined by the points in time t3 and t4 as illustrated in Fig. 4). Of course, step 320 may also be performed by the processor 230 of the device 200 accessing the corresponding temperature data in the memory 220.

Once the series of temperature measurements in the interval between skin contact and thermal equilibrium have been determined in step 320, in a further step 330 the body core temperature is determined by the processor 130 or the processor 230 from this series of temperature measurements, the associated measurement times and one or more device parameters. As has been explained above, the one or more device parameters can be retrieved from the memory 120 or the memory 220.

Body core temperature determination based on the temperature measurement data taken in any of the intervals illustrated in Fig. 4, or similar intervals, and based on the one or more device parameters can be performed in various ways. For example, a temperature change model may be applied to the measurement data to fit one or more model parameters. The body core temperature may then be determined from the model parameter(s) and the device parameter(s).

The temperature change model is in one variant tailored to reflect the temperature change characteristic illustrated in Fig. 4 between points in time t1 and t2. As such, the temperature change model may reflect a temperature growth starting at an initial temperature Ta and asymptotically approaching a final temperature, which is the skin temperature Ts. For example, the temperature change model T(t) may be expressed as being proportional to exp (- alpha * t), wherein alpha is a model parameter that can be regarded to Indicate a time behavior of the temperature change In the Interval defined by points in time t1 and t2 (in the sense of a time constant of the temperature change). As said, alpha can be determined from a fitting procedure.

When additionally knowing the thermal characteristics of the device 100, the time behavior (as indicated by the time constant alpha) of the temperature change can be exploited to determine the body core temperature. To this end, a body core temperature model Tc can be used that is parameterized in at least the time constant alpha (or parameter comprising or derived from alpha) and one or more device parameters indicative of thermal device characteristics. The body core temperature model may additionally be parameterized in one or more further model parameters derivable from the temperature growth model T(t) by fitting.

Once the body core temperature has been determined, the processor 130 or the processor 230 controls the associated display 150, 250 to display the body core temperature. Alternatively, or In addition, the body core temperature may be communicated to a central device for further processing (e.g., in a clinical environment).

After the transient phase (i.e., after the point in time t2 in Fig. 4), the measured skin temperature will remain substantially constant as illustrated In Fig. 5 until the body core temperature changes or an external event influencing the skin temperature occurs. Of course, the measured skin temperature fluctuates due to measurement artefacts even in the absence of a body core temperature change and in the absence of an external event. The measurement artefacts depend on the accuracy of the temperature sensor 110.

To prevent the measurement artefacts from influencing the determination of the body core temperature after the transient phase, the skin temperature is considered to remain constant as long as the skin temperature measurements do not fall below or exceed predefined thresholds as illustrated in Fig. 5 by dashed lines. These thresholds are set depending on the accuracy of the temperature sensor 110. As long as the skin temperature can be considered to remain constant, also the body core temperature is considered to remain constant.

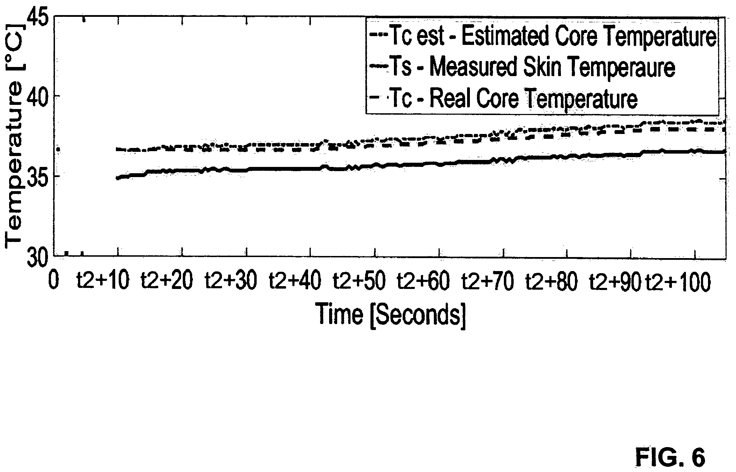

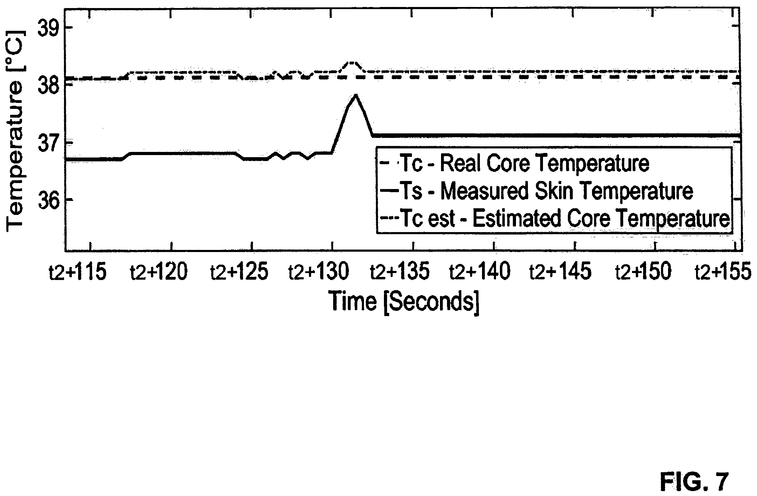

When, however, the skin temperature measurements are indicative of a temperature change that exceeds mere measurement artefacts, it has to be determined if the change is due to a body core temperature change, as illustrated in Fig. 6, or due to an external event, as illustrated in Fig. 7. As can be gathered from the continuous lines in Figs. 6 and 7, a body core temperature change leads to a slow and steady increase of the skin temperature, whereas an external event leads to a comparatively sudden skin temperature jump. In the first case, the body core temperature model parameters as determined in the transient phase before point in time t2 remain applicable for body core temperature estimation (see Fig. 6). In the second case, however, the model parameters have to be updated so that despite the skin temperature change due to the external event, the estimated body core temperature remains substantially constant (see Fig. 7).

The mere differentiation between a skin temperature change that is due to a body core temperature change on the one hand and a skin temperature change that is due to an external event can be based on looking at the magnitude of the skin temperature change in a given period of time. If the magnitude is below a predefined threshold, the skin temperature change can be attributed to a body core temperature change as shown in Fig. 6. If, on the other hand, the magnitude Is above that threshold, the skin temperature change can be attributed to an external event as shown in Fig.

7. Of course, other differentiation strategies may be used as well.

What remains a challenge is to find a proper strategy to properly detect the external event and to properly adjust the model parameters, so that despite the skin temperature change resulting from an external event, the estimated body core temperature remains substantially constant (see Fig. 7). An exemplary approach in this regard will now be explained with reference to Fig. 8.

The flow diagram of Fig. 8 Illustrates a method embodiment of determining a body core temperature from skin temperature measurements after the transient phase has ended (i.e., at or after point in time t2 in the scenario illustrated in Fig. 4). It should be appreciated that the method illustrated in Fig. 8 can be performed independently from the method illustrated in Fig. 3. This also means that the method illustrated in Fig. 3 need not necessarily be performed as a prerequisite for performing the method illustrated in Fig. 8 provided that the model parameters of the body core temperature model have been determined otherwise.

In step 810, skin temperature measurements are continued to be taken at a given sampling frequency after point in time t2. The sampling frequency after point in time t2 may be lower than the sampling frequency before point in time t2. As an example, the sampling frequency after point in time t2 may be higher by a factor of at least 10 (e.g., a new measurement may be taken every 30 seconds). In this manner, power consumption and data storage requirements can be reduced so that the device 100 can remain battery-operated when attached to the patient for hours or even days. On the other hand, the interval between two consecutive skin temperature measurements should not exceed a certain threshold (of, e.g., one or two minutes) to properly detect skin temperature changes at a sufficiently high resolution. The corresponding skin temperature measurements and associated measurement times are stored as measurement data in memory 120 or memory 130, as described above.

The skin temperature measurements taken after point in time t2 are continuously processed by the processor 130 or the processor 230 to detect a skin temperature change (as shown In Fig. 7) in step 820. To properly identify a skin temperature change due to an external event different from a body core temperature change in step 830, a temporal behavior of an average skin temperature is continuously monitored over successive points of time. As an example, a running variance or a running standard deviation may be calculated over a moving window of M skin temperature samples (e.g., M = 5, M = 10 or M = 20 or more). Of course, other statistic measures could be used as well.

Fig. 9 Illustrates in a dashed line and on a different time scale the temporal behavior of a running standard deviation for M = 10 samples in the external event scenario illustrated in Fig. 7. As explained above, the skin temperature samples may be taken at intervals of, for example, 30 seconds. In case the deviation exceeds a predefined threshold also indicated in Fig. 9, an external event is detected. The predefined threshold may be set such that skin temperature changes due to measurement artefacts (see Fig. 5) or due to body core temperature changes (see Fig. 7) remain below the threshold and, therefore, will not be identified as external events.

As long as the threshold is not trespassed by the running variance (or other statistic measure), the temperature change model and the one or more model parameters previously computed as discussed above in the context of Fig. 3 are continued to be used to determine the body core temperature (and potential changes), but on the basis of the newly taken temperature measurements and the associated measurement times (see Fig. 6). If, however, the threshold is trespassed, an external event has occurred and one or more model parameters (such as alpha or a parameter including or derived from alpha) of the body core temperature model need to be newly calculated from the skin temperature measurements underlying the external event. In this manner, the external effect can be compensated for in upcoming skin temperature measurements (step 840), assuming that the external effect will remain present and, thus, will continue to have an effect on these upcoming skin temperature measurements. For this re-calculation of the body core temperature parameter(s), the point in time associated with occurrence of the external event has to be derived first.

The temporal occurrence of the external event in the skin temperature measurement domain as Illustrated in Fig. 7 is derived from a sample corresponding to a peak location in the standard deviation domain of Fig. 9 minus M/2 samples. The peak is Identified as valid if It is detected to be located above the threshold illustrated in Fig. 9. If, for example, a valid peak Is Identified at point in time tx in the standard deviation domain and If the sampling period is n seconds, the location of the external event in the skin temperature measurement domain will be at point in time ty = tx - n * (M/2).

Then, the temperature change model as used in the transient phase is fitted to skin measurements samples before (and/or after) the point In time ty to determine a new time constant alpha1 (and/or a new time constant alpha2). As an example, 10 to 30 measurement samples before (and/or after) the point in time ty may be used for the fitting process.

The new time constant alpha1 is then used instead of the time constant alpha derived for the transient phase (see Fig. 1) for the continued body core temperature determination. Optionally, a validation of alpha1 may be made based on alpha2. If alpha1 deviates from alpha2 by more than a predefined value, then the skin temperature change is indeed due to an external event (so that alpha1 is positively validated to be useable for the continued body core temperature determination). Otherwise, i.e., if alpha1 and alpha2 do not differ significantly, the previously computed alpha value may be reused for the continued body core temperature determination.

In the following, more detailed embodiments for determining the body core temperature from measurement data taken in the transient phase (i.e., in the interval between points in time t1 and t2 in Fig. 4) and for detecting external events after the transient phase (i.e., after the point in time t2 in Fig. 4) will be presented. These embodiments may be practiced in a device scenario as illustrated in Fig. 1 or 2 and may be implemented in the context of the method embodiments illustrated in Figs. 3 and 8.

In the embodiments that follow, the heat flux from deep core body to the skin surface is modelled by the equivalent circuit illustrated in Fig. 10. In this thermal flux model, only the temperature needs to be measured by the device 100 at a series of consecutive points in time (typically regularly sampled). The remaining parameters (ambient temperature Ta, core body temperature Tc, ambient heat loss resistance Ra, ambient heat capacity Ca, and core body heat flow resistance Rc to the skin) are initially all unknown.

The thermal flux model based on the equivalent circuit illustrated in Fig. 10 encompasses a dynamic aspect to represent the temperature change (typically a growth) recorded by the temperature sensor 110 upon application of the device 100 to the skin surface 105, as generally illustrated in Fig. 4 in the interval between the points In time t1 and t2. By considering the temperature variables as equivalent to voltages and the heat flows as equivalent to currents, the thermal flux model based on the equivalent circuit illustrated in Fig. 4 can be expressed by the following equation in the Laplace domain:

$$\frac{Tc(s)-Ts(s)}{Rc} = \frac{Ts(s)-Ta(s)}{Za(s)} \qquad (1)$$

In the above equation (1), Za(s) is the impedance Ra in parallel with 1/sCa, wherein Ra represents the thermal flux resistance loss from the device 100, and the capacitor Ca represents the heat capacity of the device 100 (i.e., a device parameter indicative of a thermal device characteristic).

First assumptions can be made at this stage as follows. Over the period of observation, the ambient temperature Ta and the core body temperature Tc are substantially constant, and hence we can use their corresponding Laplace transforms Ta/s and Tc/s and obtain the system response G(s) as follows:

$$G(s) = \frac{RcTa+TcRa+sRaCaRcTa}{Ra+Rc+sRaCaRc} \qquad (2)$$

Initially, due to the heat flow from inside the body to skin and the environment, it is expected that the measured temperature will increase in some sense following skin contact until a substantially constant temperature can be measured. The associated temperature can now be considered as the output of a linear system that has as input the constants Tc and Ta which are applied simultaneously to yield as output the measurable temperature.

The time domain behavior of the temperature change can be expressed by a temperature change model having an asymptotic character as follows

$$Ts(t) = Tinf + (To - Tinf)e^{-at} \qquad (3)$$

In equation (3), the only measurements pertain to Ts(t). The model parameters Tinf (i.e., temperature measurable at t = ∞), To (i.e., temperature measureable at t = 0) and alpha (i.e., time constant "a") are unknown. Nevertheless, a nonlinear optimization procedure can be used to fit the model of equation (3) to a series of skin temperature measurements, as illustrated in Fig. 11. In MATLAB, such a fitting can be done using, inter alia, the lsqcurvefit.m function. Fig. 12 illustrates the fitting result for the skin temperature measurements of in Fig. 11.

The transfer function of the temperature growth model is then determined from Tinf(s)/(1/s) according to the following equation

$$G_{fit}(s) = \frac{aTinf+sTo}{s+a} \qquad (4)$$

The curve fitting operation now permits to determine the parameters in equations (2) and (4) as follows

$$Ta = To \qquad (5)$$

$$Tinf = \frac{TcRa + TaRc}{Ra + Rc} \quad (6)$$

$$a = \frac{Ra + Rc}{CaRaRc} \quad (7)$$

It is not surprising to find from the above equations that that the measured starting temperature of the skin (see point in time t1 in Fig. 4) is equal to the ambient temperature Ta. Equation (3) essentially enables to know the behavior of the observed variable (measured temperature) for all time. Thus, the parameters that the system illustrated in Fig. 10 would finally have under thermal equilibrium can be inferred, even though that equilibrium has not been reached.

The body core temperature Tc is obtained from a slight restructuring of equation (6) to yield a body core temperature model as expressed in equation (8) below

$$Tc[n] = Tinf[n] + \mu(Tinf[n] - Ta[n]) \quad (8)$$

In equation (8), the ambient temperature Ta is constant and Tinf can be replaced by the skin temperature data at some point, because the asymptotic behavior is attained after the transient response is complete.

The model parameter $\mu$ corresponds to Rc/Ra, which can be calculated from equation (7) by replacement with $\tau o$ = tau0 = Ca * Ra. Then, $\mu$ is estimated as illustrated by the following equation:

$$\mu = \frac{1}{\tau o a - 1} \quad (9)$$

In equation (9), the parameter $\alpha$ is obtained by a least squares curve fitting operation, while $\tau o$ = tau0 can be considered *a priori* known as a device property and can be calibrated (under the assumption that the heat flow resistance Ra is the same during calibration and actual use of the device 100). Thus, the body core temperature Tc can be derived from equations (8) and (9).

It is observed that once the transient response is finished and the equilibrium state has been reached in the skin temperature curve, the sequential window computations for $\alpha$ in equation (9) would result ultimately in values which are equal to the mean of the historical data (at a window size of, for example, M = 10). At this stage, calculation of $\mu$ is stopped and taken as a constant. The stopping point depends on the window size of the computation function in the program code. Thus, computation of $\mu$ is stopped when it Is observed that $\mu$ is no longer changing substantially (except for possible measurement artefacts as illustrated in Fig. 5). This situation will occur when the temperature has substantially reached Its asymptotic value (i.e., within about $\tau o$ seconds). Apparently, $\mu$ remains constant and can be used for determining the body core temperature as long as the parameters defining $\mu$ remain constant (in particular $\alpha$ and, thus, the ambient heat flow resistance Ra).

To determine Tc correctly, $\tau o$ should be given as an input to the computation function. In a real-world application, $\tau o$, including Ca, is derived "off line" by calibrating each device 100. This calibration can be performed as follows (e.g., at the end of a device manufacturing). For the purpose of calibration, Tc and Ta are considered to be known and constant, and Tinf and alpha0 can be determined by fitting the temperature growth model of equation (3) to the calibration temperature measurements. The parameter alpha0 indicates that it relates to an "off line" scenario.

In certain variants, it is assumed that the value of the ambient heat flow resistance during the calibration phase, Ra0, is the same as the value of the ambient heat flow resistance experienced upon an actual skin temperature measurement, Ra. This assumption is valid as long as device calibration is performed under similar covering conditions as will be encountered during the actual use of the device 100 for body core temperature determination. Since the skin will typically not be covered during use in the transient phase (as the device 100 has to be brought in direct skin contact), also the calibration phase has to be performed without covering the device 100 under calibration.

Rc/Ra can be extracted from equation (6), as in equation (10). This ratio can then be used to determine $\tau o$ for "on line" use cases, which is Ra * Ca, as in equation (11) (see also equation (7)).

$$\frac{Rc}{Ra} = \frac{Tc - Tinf}{Tinf - Ta} \quad (10)$$

$$\tau 0 = \frac{1+Rc/Ra}{a \, x \, Rc/Ra} \quad (11)$$

After the body core temperature Tc has initially been determined based on equations (8) and (9), the skin temperature measurements are continued to detect skin temperature change events. Such "events" may, for example, correspond to a jump or a gradual change in the continued skin temperature measurements and may have different reasons.

It is observed that under normal conditions it is expected to see a core body temperature variation rate of around 1.5 °C/hour. Under clinically severe cases, such as a post-surgery state, one may expect up to around 7 °C/hour. Thus, relatively sudden changes, or jumps, in the core body temperature are not expected. As a result, jumps in the measured data for a skin temperature time series will typically be the result of a change of the ambient heat flow resistance Ra. An explanation for the ambient heat flow resistance change can be an external effect different from a body core temperature change. For example, the device 100 may be covered with a blanket or a clothing layer at some point in time during the ongoing measurements, or the patient may remove a clothing layer.

Also the value of the parameter $\tau 0$ changes when changing clothing/covering layers for the following reason. As explained above, $\tau 0$ equals Ra * Ca. Upon a change of the layer(s) on top of the device 100, the ambient heat flow resistance Ra will change and, as a consequence, $\tau 0$ will change as well .In other words, the above assumption that the conditions under device calibration are the same as during use of the device 100 does no longer hold. On the other hand, such ambient changes cannot affect the value of the core body heat flow resistance Rc to skin, since this is a patient attribute. It remains substantially constant for a specific person and is related to metabolism characteristics of the person for which skin temperature measurements are taken using the device 100. Hence, by and large it remains substantially constant throughout the skin temperature measurements for a specific person.

Accordingly, when the skin temperature changes due to changing clothing/covering layers, Ra and, thus, $\tau 0$ will change also. As a consequence, the corresponding $\mu$ value changes as well for the calculation of Tc as in equation (8).

As explained above with reference to Fig. 9, the point In time associated with an external event is detected via an algorithm which looks at a running standard deviation (or variance or similar statistic measure) of a set of M samples of the continued skin temperature measurements after the point in time t2. This algorithm identifies trespassing of a standard deviation threshold set based on, for example, temperature sensor accuracy specifications. As long as the standard deviation of the continued skin temperature measurements is below that threshold, no external event is detected, so that no re-calculation of the model parameter $\mu$ is necessary and the previously calculated $\mu$ value (and the associated body core temperature model as defined in equation (8)) can further be used to determine the body core temperature from series of skin temperature measurements after point in time t2.

If, however, the threshold is trespassed, an external event has occurred that requires a re-calculation of $\mu$. In this context, the algorithm further Identifies the Instant in time at which a peak in the standard deviation domain has occurred above the threshold and makes adjustments to the time scale to line up the time of occurrence in the standard deviation domain with the associated point in time, or sample, in the skin temperature measurement domain.

Having identified the point of occurrence, or sample, in the skin temperature measurements corresponding to the peak in the standard deviation domain, the skin temperature measurements in the vicinity of that sample are evaluated to calculate a new $\alpha$ value by suitably fitting the temperature model of equation (3) to these skin temperature measurements. For example, the skin temperature measurement samples used for the fitting may start and/or end with the skin temperature measurement sample signifying the point of peak occurrence.

To calculate the new value of $\mu$ for body core temperature determination in accordance with equation (8), the following calculations of equation (12) are carried out, yielding an $\alpha$ value for $\mu$new as set out in the equation below (see also equation (7):

$$\alpha_{new} = \frac{Ra_{new}+Rc}{CaRa_{new}Rc} = \frac{Ra_{new}+Rc}{\frac{\tau 0}{Rao}Ra_{new}Rc} = \frac{Ra_{new}+Rc}{\frac{\tau 0}{\frac{Rao}{Rc}}Ra_{new}} = \frac{Ra_{new}+Rc}{\frac{\tau 0}{\mu 0}Ra_{new}} = \frac{1+\frac{Rc}{Ra_{new}}}{\tau 0 \mu 0} \quad (12)$$

Then, $\mu$new can be written as in the equation below

$$\mu_{new} = \frac{Rc}{Ra_{new}} = \alpha_{new}\mu 0 \tau 0 - 1 \quad (13)$$

Based on μnew, the body core temperature measurements are continued in accordance with equation (8) until a new external event is detected and μ has to be newly determined again. The parameter μ0 may be derived from equation (9) or otherwise.

In the following, some considerations underlying a realization of the body core temperature detection in software are given.

The program code may work as a real-time application. This effectively assumes that the skin temperature measurements are gathered in real time, one value at each sampling period of, for example 1 second during the transient phase. To fit a curve to the resulting data points, a sufficient number of measurement samples should be collected, for example between 30 and 50 or more samples as shown in Fig. 12.

To fit the temperature model, the MATLAB Isqcurvefit.m function is used to estimate the curve parameters alpha, Tinf and Ta, as explained above. The curve fitting algorithm takes the data from the starting point of 1 to the latest point. As explained above, equation (3) is used as the appropriate functional form for the curve. The parameter μ is then calculated from equation (9) in estimated steady state cases. Then the core body temperature Tc determination is done via equation (8).

Once the transient response of the data is complete (which is within around 50 data points under current sampling periods), Tinf is replaced by the current skin temperature value(s), μ is calculated from equation (9) with an average value of alpha (from 30 to 50 alpha values), and set to this constant value until an external event is detected that requires a re-calculation of μ.

For external event detection the standard deviation window is set to 10 skin temperature measurement samples at typically a higher sampling rate compared to the transient phase. Once a peak above a given threshold in the running standard deviation Is detected, then skin temperatures above the threshold may signify an ambient characteristics change as explained above and Illustrated in Fig. 9.

In this case, a new value of μ is calculated after that point. The value μnew is calculated from equation (13).

One significant advantage of the skin temperature determination approach presented herein is the fact that it can obtain all parameters needed for core body temperature determination (under the assumptions made) purely from the transient characteristics of the skin temperature measurements. The prior art approaches discussed do not Include the dynamic effect of the joint action of heat capacity and heat loss. Hence, in order to extract the required parameters they had to increase the number of sensors (thereby increasing device costs) and to wait until the measurements reached a steady temperature state before the actual body core temperature determination could start (thereby introducing significant measurement delays). Moreover, the task is much more complex when real-time tracking of changes in Tc are required after the transient phase in view if the occurrence of external events. As described above, in the exemplary embodiment such issues are obviated.

**Claims**

1. An apparatus (100, 200) for determining a body core temperature (Tc) from temperature measurements taken by a device (100) that is configured to be brought into skin contact and that Is associated with at least one device parameter (Ca) indicative of a thermal device characteristic, the apparatus being configured to:

   - determine (320) a series of temperature measurements (T(t)) taken between a first point in time when (t1) or after (t3) the device (100) is brought into skin contact and a second point in time before (t4) or when (t2) a substantially constant temperature is measured, wherein the temperature measurements (T(t)) have been taken at associated measurement times; and
   - determine (330) the body core temperature (Tc) at least from the series of temperature measurements (T(t)), the associated measurement times and the at least one device parameter (Ca).

2. The apparatus of claim 1, configured to determine (330) the body core temperature (Tc) by

   - applying a temperature change model to the series of temperature measurements (T(t)) and the associated measurement times to determine a model parameter (alpha) indicative of a time behavior of the temperature change; and
   - determining the body core temperature (Tc) at least from the model parameter (alpha) and the device parameter (Ca).

3. The apparatus of claim 2, configured to

   further determine at least one of an initial temperature parameter (T0) and a final temperature parameter (Tinf) from the temperature change model and to determine the body core temperature (Tc) also from at least one of the initial temperature parameter (T0) and the final temperature parameter (Tinf), wherein the initial temperature parameter (T0) is indicative of a temperature that would be measurable at t = 0 before the device is brought into skin contact and the final temperature parameter (Tinf) is indicative of a temperature that would be measureable at t = ∞.

4. The apparatus of claim 3, wherein

the temperature change model In a time domain representation Is based on the following expression:

$$T(t) = Tinf + (T0 - Tinf) * exp(-alpha * t),$$

wherein T(t) is the temperature measurement at measurement time t, alpha is the model parameter indicative of the time behavior of the temperature change, T0 is the initial temperature parameter and Tinf is the final temperature parameter.

5. The apparatus of claim 3 or 4, configured to

determine the body core temperature (Tc) based on the following expression:

$$Tc = f(Ca; alpha; T0; Tinf),$$

wherein Ca is the device parameter, alpha Is the model parameter Indicative of the time behavior of the temperature change, T0 is the Initial temperature parameter and Tinf is the final temperature parameter.

6. The apparatus of any of any of the preceding claims, configured to

- process continued temperature measurements taken after the second point in time (t2) to detect a skin temperature change; and
- update the body core temperature (Tc) on the basis of the continued temperature measurements comprising the detected skin temperature change.

7. The apparatus of claim 6, configured to

determine If the detected skin temperature change is due to an external effect different from a body core temperature change.

8. The apparatus of claim 7, configured to

evaluate a temporal behavior of the skin temperature change to determine if the detected skin temperature change is due to an external effect.

9. The apparatus of claim 8, configured to

determine that the detected skin temperature change is due to an external effect if a magnitude of the skin temperature change within a given period of time exceeds a predefined temperature change threshold.

10. The apparatus of any of claims 7 to 9 in combination with any of claims 2 to 5, configured to

newly calculate at least one parameter of the temperature change model if the detected skin temperature change is due to an external effect.

11. The apparatus of claim 10, configured to

newly determining at least one parameter of the temperature change model by applying the temperature change model to at least some of the skin temperature measurements from which the external effect has been determined.

**12.** The apparatus of any of claims 7 to 9 in combination with any of claims 2 to 5, configured to

re-use, for determining the body core temperature from the continued skin temperature measurements, the at least one previously determined parameter of the temperature change model If the detected skin temperature change is not due to an external effect.

**13.** The apparatus of any of the preceding claims, configured to

detect that the device has been brought into skin contact.

**14.** The apparatus of any of the preceding claims, wherein

the device parameter (Ca) has been obtained in a calibration procedure preceding the temperature measurements.

**15.** The apparatus of claim 14, wherein

the calibration procedure comprises measuring, by the device, a calibration temperature of a calibration body, wherein the calibration temperature or a temperature difference between the calibration temperature and ambient temperature is known *a priori*.

**16.** A method of determining a body core temperature (Tc) from temperature measurements taken by a device (100, 200) associated with at least one device parameter (Ca) indicative of a thermal device characteristic, the method comprising:

- determining (320) a series of temperature measurements (T(t)) taken between a first point in time when (t1) or after (t3) the device (100) is brought into skin contact and a second point in time before (t4) or when (t2) a substantially constant temperature is measured, wherein the temperature measurements (T(t)) have been taken at associated measurement times; and
- determining (330) the body core temperature (Tc) at least from the series of temperature measurements (T(t)), the associated measurement times and the at least one device parameter (Ca).

**17.** An apparatus (100, 200) for processing skin temperature measurements to determine a body core temperature (Tc) from a body core temperature model, the apparatus being configured to:

- process (820) a series of skin temperature measurements (T(t)) taken at associated measurement times to detect a skin temperature change;
- determine (830) that the detected skin temperature change is due to an external effect different from a body core temperature change; and
- determine (840) at least one model parameter ($\mu$) of the body core temperature model so as to compensate for the external effect when determining the body core temperature from further skin temperature measurements T(t).

**18.** The apparatus of claim 17, configured to

determine the at least one model parameter ($\mu$) by applying a temperature change model to the series of skin temperature measurements from which the external effect was determined.

**19.** The apparatus of claim 18, configured to

determine a point in time (ty) indicative of occurrence of the external event in the series of skin temperature measurements and apply the temperature change model to a sub-series of the skin temperature measurements preceding that point in time (ty).

**20.** A method for processing skin temperature measurements to determine a body core temperature (Tc) from a body core temperature model, the method comprising:

- processing (820) a series of skin temperature measurements (T(t)) taken at associated measurement times to detect a skin temperature change;
- determining (830) that the detected skin temperature change is due to an external effect different from a body core temperature change; and
- determining (830) at least one model parameter ($\mu$) of the body core temperature model so as to compensate for the external effect when determining the body core temperature from further skin temperature measurements T(t).

21. A computer program product comprising program code portions to perform the method of claim 16 or 20 when the computer program product is executed by at least one processor.

**Amended claims in accordance with Rule 137(2) EPC.**

1. An apparatus (100, 200) for determining a body core temperature (Tc) from temperature measurements taken by a device (100) that is configured to be brought into skin contact and that is associated with at least one device parameter (Ca) indicative of a thermal device characteristic, the apparatus being configured to:

- determine (320) a series of temperature measurements (T(t)) taken between a first point in time when (t1) or after (t3) the device (100) is brought into skin contact and a second point in time before (t4) or when (t2) a substantially constant temperature is measured, wherein the temperature measurements (T(t)) have been taken at associated measurement times; and
- determine (330) the body core temperature (Tc) at least from the series of temperature measurements (T(t)), the associated measurement times and the at least one device parameter (Ca).

2. The apparatus of claim 1, configured to determine (330) the body core temperature (Tc) by

- applying a temperature change model to the series of temperature measurements (T(t)) and the associated measurement times to determine a model parameter (alpha) indicative of a time behavior of the temperature change; and
- determining the body core temperature (Tc) at least from the model parameter (alpha) and the device parameter (Ca).

3. The apparatus of claim 2, configured to further determine at least one of an initial temperature parameter (T0) and a final temperature parameter (Tinf) from the temperature change model and to determine the body core temperature (Tc) also from at least one of the initial temperature parameter (T0) and the final temperature parameter (Tinf), wherein the initial temperature parameter (T0) is indicative of a temperature that would be measurable at t = 0 before the device is brought into skin contact and the final temperature parameter (Tinf) is indicative of a temperature that would be measureable at t = $\infty$.

4. The apparatus of claim 3, wherein the temperature change model in a time domain representation is based on the following expression:

$$T(t) = Tinf + (T0 - Tinf) * \exp(-alpha * t),$$

wherein T(t) is the temperature measurement at measurement time t, alpha is the model parameter indicative of the time behavior of the temperature change, TO is the initial temperature parameter and Tinf is the final temperature parameter.

5. The apparatus of claim 3 or 4, configured to determine the body core temperature (Tc) based on the following expression:

$$Tc = f(Ca; alpha; T0; Tinf),$$

wherein Ca is the device parameter, alpha is the model parameter indicative of the time behavior of the temperature change, TO is the initial temperature parameter and Tinf is the final temperature parameter.

6. The apparatus of any of any of the preceding claims, configured to

    - process continued temperature measurements taken after the second point in time (t2) to detect a skin temperature change; and
    - update the body core temperature (Tc) on the basis of the continued temperature measurements comprising the detected skin temperature change.

7. The apparatus of claim 6, configured to
   determine if the detected skin temperature change is due to an external effect different from a body core temperature change.

8. The apparatus of claim 7, configured to
   evaluate a temporal behavior of the skin temperature change to determine if the detected skin temperature change is due to an external effect.

9. The apparatus of claim 8, configured to
   determine that the detected skin temperature change is due to an external effect if a magnitude of the skin temperature change within a given period of time exceeds a predefined temperature change threshold.

10. The apparatus of any of claims 7 to 9 in combination with any of claims 2 to 5, configured to
    newly calculate at least one parameter of the temperature change model if the detected skin temperature change is due to an external effect.

11. The apparatus of claim 10, configured to
    newly determining at least one parameter of the temperature change model by applying the temperature change model to at least some of the skin temperature measurements from which the external effect has been determined.

12. The apparatus of claim 11, configured to
    determine a point in time (ty) indicative of occurrence of the external event in the series of skin temperature measurements and apply the temperature change model to a sub-series of the skin temperature measurements preceding that point in time (ty).

13. The apparatus of any of claims 7 to 9 in combination with any of claims 2 to 5, configured to
    re-use, for determining the body core temperature from the continued skin temperature measurements, the at least one previously determined parameter of the temperature change model if the detected skin temperature change is not due to an external effect.

14. The apparatus of any of the preceding claims, configured to
    detect that the device has been brought into skin contact.

15. The apparatus of any of the preceding claims, wherein
    the device parameter (Ca) has been obtained in a calibration procedure preceding the temperature measurements.

16. The apparatus of claim 15, wherein
    the calibration procedure comprises measuring, by the device, a calibration temperature of a calibration body, wherein the calibration temperature or a temperature difference between the calibration temperature and ambient temperature is known *a priori.*

17. A method of determining a body core temperature (Tc) from temperature measurements taken by a device (100, 200) associated with at least one device parameter (Ca) indicative of a thermal device characteristic, the method comprising:

    - determining (320) a series of temperature measurements (T(t)) taken between a first point in time when (t1) or after (t3) the device (100) is brought into skin contact and a second point in time before (t4) or when (t2) a substantially constant temperature is measured, wherein the temperature measurements (T(t)) have been taken at associated measurement times; and
- determining (330) the body core temperature (Tc) at least from the series of temperature measurements (T(t)), the associated measurement times and the at least one device parameter (Ca).

18. A computer program product comprising program code portions to perform the method of claim 17when the computer program product is executed by at least one processor.

Ambient Temperature Ta

**Measuring and Processing Device 100**

Display 150

Transceiver 140

Processor 130

Memory 120

Skin 105
(Skin Temperature Ts)

Temperature Sensor 110

Body Core Temperature Tc

**FIG. 1**

EP 3 505 891 A1

Ambient Temperature Ta

**FIG. 2**

Skin 105
(Skin Temperature Ts)

Body Core Temperature Tc

EP 3 505 891 A1

FIG. 3

FIG. 4

FIG. 5

FIG. 6

**FIG. 7**

Europäisches
Patentamt

European
Patent Office

Office européen
des brevets

## EUROPEAN SEARCH REPORT

Application Number

EP 17 39 0001

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>Y | US 2005/043631 A1 (FRADEN JACOB [US])<br>24 February 2005 (2005-02-24)<br>* abstract; figures 4,6,9 *<br>* paragraphs [0026] - [0036] * | 1-6,<br>13-16<br>7-12 | INV.<br>G01K7/42<br>A61B5/01<br>G01K13/00 |
| A | WO 2017/108459 A1 (KONINKLIJKE PHILIPS NV [NL]) 29 June 2017 (2017-06-29)<br>* abstract; figures 1,3 *<br>* page 5, line 15 - page 9, line 19 * | 1-6,16 | |
| A | US 2007/282218 A1 (YARDEN MOSHE [IL])<br>6 December 2007 (2007-12-06)<br>* abstract; figure 3 *<br>* paragraphs [0063] - [0072], [0084] - [0094] * | 1,6,16 | |
| X<br>Y | US 8 930 147 B2 (PRIMA-TEMP INC)<br>6 January 2015 (2015-01-06)<br>* abstract; figures 4-8 *<br>* column 10, line 45 - column 13, line 26 * | 17-21<br>7-12 | |
| A | GB 2 286 684 A (WENLOCK JANETTE MARIE [GB]) 23 August 1995 (1995-08-23)<br>* abstract; figure 1 *<br>* page 7 * | 17-21 | TECHNICAL FIELDS SEARCHED (IPC)<br><br>G01K<br>A61B |

The present search report has been drawn up for all claims

| Place of search | Date of completion of the search | Examiner |
|---|---|---|
| The Hague | 22 November 2018 | de Bakker, Michiel |

EPO FORM 1503 03.82 (P04C01)

Europäisches
Patentamt

European
Patent Office

Office européen
des brevets

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☒ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION
SHEET B**

Application Number

EP 17 39 0001

The Search Division considers that the present European patent application does not comply with the requirements of unity of invention and relates to several inventions or groups of inventions, namely:

1. claims: 1-16

   Determining body core temperature from a series of temperature measurements and a device parameter

   ---

2. claims: 17-21

   Compensating for external effects on body core temperature determination

   ---

## ANNEX TO THE EUROPEAN SEARCH REPORT
## ON EUROPEAN PATENT APPLICATION NO.

EP 17 39 0001

This annex lists the patent family members relating to the patent documents cited in the above-mentioned European search report.
The members are as contained in the European Patent Office EDP file on
The European Patent Office is in no way liable for these particulars which are merely given for the purpose of information.

22-11-2018

| Patent document cited in search report | | Publication date | Patent family member(s) | | Publication date |
|---|---|---|---|---|---|
| US 2005043631 | A1 | 24-02-2005 | CN | 101031233 A | 05-09-2007 |
| | | | EP | 1768546 A1 | 04-04-2007 |
| | | | JP | 4824020 B2 | 24-11-2011 |
| | | | JP | 2008502903 A | 31-01-2008 |
| | | | US | 2005043631 A1 | 24-02-2005 |
| | | | US | 2007100564 A1 | 03-05-2007 |
| | | | WO | 2006009585 A1 | 26-01-2006 |
| WO 2017108459 | A1 | 29-06-2017 | CN | 108431566 A | 21-08-2018 |
| | | | EP | 3394583 A1 | 31-10-2018 |
| | | | WO | 2017108459 A1 | 29-06-2017 |
| US 2007282218 | A1 | 06-12-2007 | NONE | | |
| US 8930147 | B2 | 06-01-2015 | US | 2011213559 A1 | 01-09-2011 |
| | | | US | 2015297146 A1 | 22-10-2015 |
| GB 2286684 | A | 23-08-1995 | NONE | | |

EPO FORM P0459

For more details about this annex : see Official Journal of the European Patent Office, No. 12/82

## REFERENCES CITED IN THE DESCRIPTION

**Non-patent literature cited in the description**

- **SOLMAN et al.** New thermometers for deep tissue temperature. *Biomedical Engineering,* October 1973, 432-435
- **FOX.** A new technique for monitoring deep body temperature from the skin surface. *Clin. Sci.,* 1973, vol. 44, 81-86
- **KITAMURA et al.** Development of a new method for the noninvasive measurement of deep body temperature without a heater. *Medical Engineering & Physics,* 2010, vol. 32, 1-6